# EUROPEAN PATENT APPLICATION

(11) **EP 2 789 605 A1**
(43) Date of publication of application: **15.10.2014**
(21) Application number: 14158772.5
(22) Date of filing: 11.03.2014
(51) Int. Cl.: C07D 313/12, C07D 337/12

(54) **Process for the preparation of pharmaceutical intermediates**

(30) Priority: 11.04.2013 IT MI20130585
(71) Applicant: Dipharma Francis S.r.l., 20021 Baranzate (MI) (IT)
(72) Inventor: Artico, Marco, 20021 Baranzate (IT); Attolino, Emanuele, 20021 Baranzate (IT)
(74) Representative: Rezoagli, Eleonora

(57) **Abstract**

Process for the preparation of dibenzo[b,e]oxe-/thiepin-11-(6H9-one compounds that are useful intermediates in the synthesis of known tricycli antidepressants Doxepin and Dothiepin respectively.

## Description

### FIELD OF THE INVENTION

The present invention relates to a process for preparing intermediates useful in the synthesis of active pharmaceutical ingredients.

### BACKGROUND ART

Dibenzo[b,e]oxepin-11-(6H)-one, commonly known as dibenzoxepinon of formula **(A)** and dibenzo[b,e]thiepin-11-(6H)-one of formula **(B)** 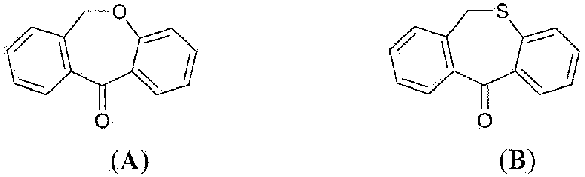 are highly interesting intermediates since they are key intermediates in the synthesis of tricyclic antidepressant compounds such as Doxepin and Dothiepin.

Dibenzoxepinon of formula **(A)** is commonly prepared by intramolecular cyclization of 2-(fenoxymethyl)benzoic acid of formula (C) or better, of an activated derivative thereof, such as the corresponding acid chloride. 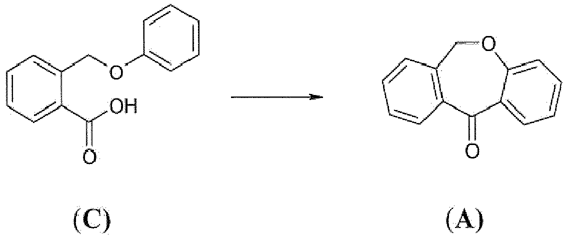 This because of the low reactivity of carboxylic acids in Friedel Crafts reactions; whereas the corresponding acid chloride behaves as a good acylating agent. The activation of the acyl chloride of a compound of formula (C), to obtain dibenzoxepinon, requires at the same the use of at least stoichiometric amounts of Lewis acids, commonly AlCl₃, used as activating agents in the Friedel-Crafts reaction. For this reason, to obtain an efficient, cheap and sustainable process, it should be desirable using activating systems in catalytic amounts.

Notwithstanding the relevant efforts made during the years to render Friedel-Crafts reaction more efficient and eco-friendly, nowadays the cheaper industrial processes, besides making use, in any case, of Lewis acids as activating agents in more than stoichiometric amounts, also make use of relevant amounts of chlorinated solvents, such as dichloromethane, dichloroethane or chlorobenzenes.

The preparation of dibenzo[b,e]thiepin-11-(6H)-one of formula **(B)** is disclosed for example in US 3,527,766, wherein it is carried out by cyclization at 140°C of 2-(phenylthiomethyl)benzoic acid in polyphosphoric acid (PPA), used in more than stoichiometric amounts, both as activating agent and reaction solvent.

Even if PPA is a good solvent for organic compounds, it can be used at high temperatures and is not so much acid, it is nevertheless a highly viscous and hygroscopic liquid. Furthermore, at the end of the reaction, in order to recover the desired product it is necessary to dilute PPA with great amounts of water and, before discharging the refluents, and neutralize the acidity by adding a suitable base with formation of great amounts of salts which are then be digested.

For these reasons there is still the need of providing an industrial process for preparing dibenzoxepinon or dibenzo[b,e]thiepin-11-(6H)-one, which makes use of lower amounts of non-toxic and potentially carcinogenic solvents, as chlorinated solvents are, and catalytic amounts of activating agents. The process should also be economical, both from point of view of the reagents and the treatment of the refluents. If necessary the process should also allow an easy recycling of the solvents and/or of the activating reagent used in the cyclization. Furthermore the method should foresee mild reaction conditions and, at the same time, provide the desired compound in high yield.

### SUMMARY OF THE INVENTION

The invention provides an alternative and improved process for preparing dibenzoxepinon and dibenzo[b,e]thiepin-11-(6H)-one, having formula **(I),** reported here below, comprising cyclizing an acid chloride of formula **(II),** as herein defined, in the presence of catalytic amounts of FeCl₃, and the use thereof in preparing Doxepin e Dothiepin, respectively.

### DETAILED DESCRIPTION OF THE INVENTION

The object of the present invention is a process for the preparation of a compound of formula (**I**): 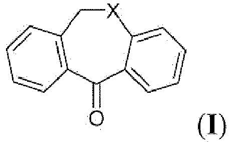 wherein X is O or S, comprising cyclizing a compound of formula **(II):** 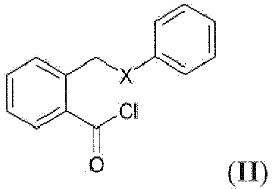 wherein X is as defined above, in the presence of a catalytic amount of FeCl₃.

A catalytic amount of FeCl₃ is typically comprised between about 0.05% molar and about 10% molar, preferably between about 2% molar and about 8% molar.

If the case, the reaction can be carried out in a solvent, in particular in the presence of low volumes of a solvent, typically two or three volumes thereof. Such solvent can be then recycled by distillation at the end of the reaction.

A solvent is typically an organic apolar aprotic solvent having a boiling point comprised between about 50°C and about 150°C, typically a straight or branched C₆-C₁₂ alkane, a C₅-C₇ cyclic hydrocarbon, an aromatic hydrocarbon, such as toluene, or a mixture for example of two of them.

Preferably the solvent is heptane, cyclohexane or toluene; more preferably toluene.

According to another embodiment of the invention, the cyclization reaction can be carried out without the use of any solvent, for example by melting the compound of formula (**II**) and adding a catalytic amount of FeCl₃.

The cyclization reaction can be carried out in a temperature range comprised between about 0°C and the mixture reflux temperature.

In particular, when the starting compound is a compound of formula formula (**II**) wherein X is oxygen, the cyclization is preferably carried out at a temperature comprised between about 0° and about 40°C; when the starting product is a compound of formula (**II**) wherein X is sulphur, the cyclization is preferably carried out at a temperature comprised between about 60° and the reflux temperature of the reaction mixture.
At the end of the cyclization reaction the so obtained mixture can be subjected to end-reaction aqueous treatments, well known to the man skilled in the art, to remove the iron salts, and the so obtained crude product of formula **(I)** can be extracted in a solvent and crystallized according to known methods.

The compound of formula **(I),** synthesized with the process of the present invention, can be obtained with a reaction yield evaluated by HPLC, equal to or higher than 90%, and, after purification by crystallization, can be obtained with a yield higher than 70% and a purity equal to or higher than 98%, preferably equal to or higher than 99%.

FeCl₃ is a known Lewis acid used in Friedel-Crafts acylation reactions in stoichiometric or sub-stoichiometric amounts, in any case higher than molar 10% compared to the starting substrate. Prior to the present invention in this reaction FeCl₃ has never been used in catalytic amounts, therefore its use very surprisingly has been found to result in the formation of the desired products in a so high yield.

Therefore, a compound of formula **(I),** prepared according to the process of the invention, is a product with such a quality to be particularly suitable as intermediate in the preparation of high quality API (Active Pharmaceutical Ingredient), in particular active on the central nervous system (SNC), such as for example Doxepin o Dothiepin.

The compound of formula (**II**) can be prepared starting from a corresponding carboxylic acid of (**III**): 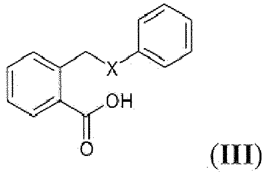 wherein X is as defined above, for example, by treatment with SOCl₂, if the case in the presence of a solvent.

The reaction of a compound of formula (**III**) with SOCl₂ can be carried out according to known methods.

According to a preferred embodiment of the present invention, the end-reaction mixture, containing a so obtained compound of formula (**II**), can be used out without additional treatments, adding a catalytic amount of FeCl₃, as reported above, in the same reactor, namely making a "one-pot reaction", so as to obtain a compound of formula (**I**) in two synthetic steps.

According to another embodiment of the invention, the reaction of a compound of formula (**III**) with SOCl₂ to obtain a compound of formula **(II)** and its cyclization to obtain a compound of formula (**I**) can be carried out substantially at the same time, treating a compound of formula (**III**) with SOCl₂ and a catalytic amount of FeCl₃, optionally in the presence of a solvent as defined above.

With the term "substantially simultaneously" the prompt subsequent or the contemporaneous cyclization reaction is intended.

Therefore, a further object of the invention is the preparation of a compound of formula **(I)** by a process comprising the conversion of the acid of formula (**III**) to a chloride of formula (**II**) and its substantially simultaneous cyclization in the presence of a catalytic amount of FeCl₃, in case in the presence of a solvent.

The compounds of formula (**I**) prepared according to the present invention, and obtained in a so high chemical purity, can be respectively converted intto Doxepin and Dothiepin, according to known methods. For example, such conversion can be carried out by a reaction comprising the reaction of a compound of formula (**I**), wherein X is O or S, with 3-dimethylaminopropylmegnesium chloride to obtain the corresponding tertiary alcohol and the subsequent treatment with an acid, for example sulfuric acid, analogously to what is reported in Example 2 of US 3,527,766.

It is therefore object of the present invention a compound of formula **(I),** wherein X is as defined above, as obtainable according to the process of the present invention, having a purity equal to or higher than 98%, preferably equal to or higher than 99%.

Furthermore, it is a further object of the present invention a process for the preparation of Doxepin or Dothiepin, comprising using a compound of formula (**I**), wherein X is O or S respectively, as starting material obtained according to the process of the invention.

Preferably, so obtained Doxepin and Dothiepin have a purity higher than 99.5%, more preferably higher than 99.8%.

The following example illustrate the invention:

### Example: Preparation of dibenzo[b,e]oxepin-11-(6H)-one (I; X=O)

In a 500 mL poly-necked flask with mechanical stirrer, thermometer, refrigerant, in N₂ atmosphere, 96% 2-(phenenoxymethyl)benzoic acid **(III)** (26 g, 109 mmol) and thyonyl chloride (26 mL) are loaded. The mixture is stirred and heated at 50°C for 2 hours, then the exceeding thionyl chloride is distilled off under reduced pressure. The temperature of the residue is brought to 25°C and 50 mL of toluene and FeCl₃ (0.80 g, 6 mmol) are added. The reaction mixture is maintained under stirring for 10 hours at about 25°C and then treated with H₂O (20 mL), and the phases are separated. The organic phase is in addition washed with H₂O, and then concentrated under reduced pressure. The so obtained residue is taken up with isopropanol.

The mixture is heated at 60°C, then maintained under stirring and slowly cooled to 5°C. The crystallized solid is filtered on Buchner, washed with isopropanol and dried in oven at 50°C under vacuum till a constant weight is obtained. This way 17.2 g of dibenzoxepinon (**I**) are obtained with a yield of 75% and a purity of 99.3% measured by HPLC.

¹H-NMR, (CDCl₃) δ: 8.24 (dd, *J* = 8.4, *J* = 2.1 Hz, 1H), 7.90 (dd, *J=* 7.5, *J=* 1.5 Hz, 1H), 7.55-7.45 (m, 3H), 7.35 (d, *J=* 7.5 Hz, 1H), 7.12 (dt, *J*= 8.4, *J*=0.9 Hz, 1H), 7.05 (dd, *J*= 8.4, *J* = 0.9 Hz, 1H), 5.19 (s, 2H).

In a same manner and using 2-(phenylthiomethyl)benzoic acid as starting material, dibenzo[b,e]thiepin-11-(6H)-one can be obtained.

## Claims

1. A process for preparing a compound of formula **(I):** 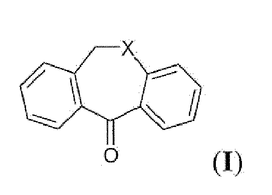 wherein X is O or S, comprising cyclizing a compound of formula (**II**): 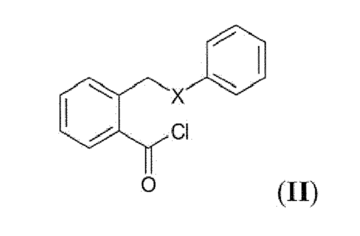 wherein X is as defined above, in the presence of a catalytic amount of FeCl₃.

2. The process according to claim 1, wherein the catalytic amount of FeCl₃ is comprised between about 0.05% and 10% molar, preferably between about 2% and 8% molar.

3. The process according to claim 1, wherein the reaction is carried out in a solvent, preferably in an amount of two or three volumes.

4. The process according to claim 3, wherein the solvent is an apolar aprotic organic solvent having a boiling point comprised between about 50°C and about 150°C.

5. The process according to claim 4, wherein the solvent is selected from the group comprising a straight or branched C₆-C₁₂ alkane, a cyclic C₅-C₇ hydrocarbon, an aromatic hydrocarbon and a mixture for example of two of them; preferably beptane, cyclohexane or toluene; more preferably is toluene.

6. The process according to claim 1, wherein the reaction is carried out without the use of any solvent, preferably by melting a compound of formula (**II**) and adding FeCl₃.

7. The process according to claims 1, 2, 3 or 6, wherein the reaction is carried out in a range of temperature comprised between about 0°C and the reflux temperature of the reaction mixture.

8. The process according to claim 7, wherein, when in the compound of formula (**II**) X is O, the reaction is carried out at a temperature comprised between about 0° and about 40°C; and, when in the compound of formula (**II**) X is S, the reaction is carried out at a temperature comprised between about 60° and the reflux temperature of the reaction mixture.

9. The process according to claims 1, 2, 3, 6 or 7, comprising preparing a compound of formula (**II**) from the corresponding carboxylic acid of formula (**III**): 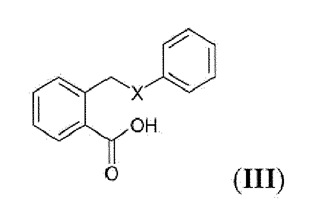 wherein X is as defined in claim 1, in case in the presence of a solvent, and cyclizing it by adding a catalytic amount of FeCl₃ in the same reactor; or comprising the contemporaneous conversion of a compound of formula (**III**) into a compound formula (**II**) and cyclizing it by adding a catalytic amount of FeCl₃, in case in the presence of a solvent.

10. A process for preparing Doxepin or Dotiepin comprising utilizing, as starting material, a compound of formula (**I**), wherein X is O or S respectively, obtained according to the process of each of preceding claims 1-9.
